# EUROPEAN PATENT APPLICATION

(11) **EP 1 600 162 A1**
(43) Date of publication of application: **30.11.2005**
(21) Application number: 05107763.4
(22) Date of filing: 21.12.1999
(51) Int. Cl.: A61K 38/26, A61K 38/28, A61K 47/10, A61K 47/26, A61P 3/10

(54) **Shelf-stable formulation of glucagon-like peptide-1**

(30) Priority: 22.12.1998 US 113499 P
(62) Divisional of application: 99967463.3
(71) Applicant: Eli Lilly & Company, Indianapolis, IN 46285 (US)
(72) Inventor: Brader, Mark Laurence, 46220, Indianapolis (US); Pekar, Allen Howard, 46220, Indianapolis (US)
(74) Representative: Kent, Lindsey Ruth

(57) **Abstract**

Glucagon-like peptide-1 (GLP-1) has been shown to be useful in the treatment of diabetes. The invention encompasses a shelf stable formulation that comprises a therapeutically effective amount of GLP-1, a pharmaceutically acceptable preservative, and a tonicity modifier, and that has a pH between about 8.2 to about 8.8.

SEQUENCE LISTING

<110> Brader, Mark L.
Pekar, Allen H.

<120> SHELF-STABLE FORMULATION OF GLUCAGON-LIKE PEPTIDE-1

<130> X-11869A

<140> US 09/868,974
<141> 1999-12-21

<150> PCT/US99/30395
<151> 1999-12-21

<150> US 60/113,499
<151> 1998-12-22

<160> 3

<170> PatentIn version 3.1

<210> 1
<211> 31
<212> PRT
<213> Artificial Sequence

<220>
<223> synthetic construct
<220>
<221> MISC_FEATURE
<222> (2)..(2)
<223> Xaa at position 2 is Ala, Gly or Val

<220>
<221> MISC_FEATURE
<222> (15)..(15)
<223> Xaa at position 15 is Glu or Gln

<220>

<221>  MISC_FEATURE

<222>  (21)..(21)

<223>  Xaa at position at 21 is Glu or Gln


<400>  1

His Xaa Glu Gly Thr Phe Thr Ser Asp Val Ser Ser Tyr Leu Xaa Gly
1               5                   10                  15

Gln Ala Ala Lys Xaa Phe Ile Ala Trp Leu Val Lys Gly Arg Gly
            20                  25                  30

<210>  2

<211>  29

<212>  PRT

<213>  Artificial Sequence


<220>

<223>  synthetic construct

<220>

<221>  MISC_FEATURE

<222>  (29)..(29)

<223>  Xaa at position 29 is Gly or is absent


<400>  2

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Xaa
            20                  25

<210>  3

<211>  30

<212>  PRT

<213>  Artificial Sequence


<220>

<223>  synthetic construct

<220>

<221>  MISC_FEATURE

<222>  (19)..(19)

<223>  Xaa at position 19 is Lys or Arg

<220>

<221>  MISC_FEATURE

<222>  (30)..(30)

<223>  Xaa at position 30 is Gly or is absent


<400>  3

Ala Glu Gly Thr Phe Thr Ser Asp Val Ser Ser Tyr Leu Glu Gly Gln
1               5                   10                  15

Ala Ala Xaa Glu Phe Ile Ala Trp Glu Val Lys Gly Arg Xaa
            20                  25                  30

## Description

### Background of the Invention

Glucagon-like peptide-1 (7-37)-OH (GLP-1) is a 31 amino acid hormone that is produced by post-translational processing of the preglucagon gene product in the brain, stomach, intestine, and pancreas. The main physiological function of GLP-1 is to regulate insulin secretion in response to glucose, and thus it has the ability to normalize blood glucose levels. As such, there has been interest in GLP-1, its analogs and derivatives as potential therapeutic agents for the treatment of diabetes. A particular advantage to the use of GLP-1 over other drugs in the treatment of diabetes is that administration of GLP-1 at doses in the 1-5 nmole range exhibit few adverse side effects, such as hypoglycemia. Unexpectedly, GLP-1 also has been shown to work in patients that have secondary failure to sulfonylurea drugs, the most common drug type for the treatment of type II diabetes. GLP-1 also is a potent inhibitor of gastric acid secretion and gastric emptying.

In general, effective therapeutic administration of peptides can be problematic since peptides often are degraded in the gastrointestinal tract by various peptidases. Additionally, certain peptide treatment protocols require either continuous or repeated administration of the peptide agent over an extended period of time. Repeated injections cause both inconvenience and discomfort to the user. Thus, chronic use of the peptide agent, which would be required for patients afflicted with diabetes, would result in inconvenience and discomfort to the user.

The long-term stability of peptides, particularly GLP-1, as components of a pharmaceutical composition for administration to mammals, is questionable. Such a lack of stability adversely affects bioavailability. In fact, when stored at low temperatures of 4° C, by-products of GLP-1(7-37) have been found as early as eleven months after sample preparation (see Mojsov, *Int. J. Peptide Protein Res.,* Vol. 40, pages 333-343 (1992)). Additionally, the biological half-life of GLP-1 molecules, particularly those molecules affected by the activity of dipeptidyl-peptidase IV (DPPIV), is quite short. For example, the biological half-life of GLP-1(7-37) is only 3 to 5 minutes (see U.S. Patent No. 5,118,666), which is further augmented by its rapid absorption following parenteral administration to a mammal.

Another factor decreasing the bioavailability of GLP-1 is the solubility of GLP-1 when incorporated into an aqueous solution. The solubility of GLP-1 is highly dependent on the environment, such as the choice of buffering system, and the treatment that the peptide has undergone. For example, conversion of a peptide into its salt form plays a role in its solubility. In this regard, synthetic GLP-1 is highly soluble in neutral phosphate buffered saline. Because the solubility of the peptide is high in such aqueous solutions, slow release of the peptide can be difficult to attain unless the peptide is incorporated into a system for slow release.

Stable formulations of therapeutic agents are particularly required for use in delivery devices that expose these agents to elevated temperatures and/or mechanical stress. For example, stable GLP-1 formulations are required for use in continuous infusion systems and pen delivery devices. Current formulations provide only limited stability in these types of delivery devices.

In continuous infusion systems, a fluid containing a therapeutic agent is pumped from a reservoir, usually to a subcutaneous, intravenous, or intraperitoneal depot. The reservoir, which must be refilled periodically, is attached to the patient's body, or is implanted in the patient's body. In either case, the patient's body heat and body motion, and turbulence in the tubing and pump impart a relatively high amount of thermomechanical energy to the formulation. In the interest of minimizing the frequency with which the reservoir is refilled, and of minimizing the size of the reservoir, formulations having a relatively high concentration of the therapeutic agent are advantageous.

Injector pens also have been developed to allow diabetic patients to accurately measure and administer controlled doses of insulinotropic agents. Generally, these pens are secured onto a cartridge having a particular quantity of liquid medication sealed therein. The cartridge includes a plunger and a mechanism for advancing the plunger in the cartridge in such a manner to dispense the medication. Injector pens may be reusable or disposable. In reusable pens, a user can change a spent cartridge and reset the leadscrew of the pen back to its initial position. In a disposable pen, the cartridge is permanently captured in the pen which is disposed of after the contents of the cartridge have been exhausted.

With the development of GLP-1 as well as analogs and derivatives thereof for the treatment of diabetes, there exists a need to improve treatment regimes that can balance chemical and physical stability with chronic use requirements of diabetic patients.

### Summary of the Invention

In order to overcome the problems of chemical and physical stability of GLP-1 formulations, the present inventors have developed a shelf-stable formulation of GLP-1. In particular, the inventors have discovered that when certain physiologically tolerated buffers are used in formulations of GLP-1, the physical stability of such formulations is unexpectedly and considerably greater than when compared to GLP-1 formulations made with a phosphate buffer. In addition, maintaining the pH in a range of about 8.2 to about 8.8 unexpectedly improves the chemical stability of the formulations. The shelf-stable formulation of GLP-1 of the invention comprises a therapeutically effective amount of a GLP-1 molecule, a pharmaceutically acceptable preservative, and a tonicity modifier, wherein the pH of said formulation is maintained in the range from about 8.2 to about 8.8.

In accordance with the chemical and physical stability needs of GLP-1 formulations, the present invention provides a shelf-stable pharmaceutical formulation comprising a therapeutically effective amount of a GLP-1 molecule, a pharmaceutically acceptable preservative, and a tonicity modifier, wherein the formulation has a pH that is about 8.2 to about 8.8. In a preferred embodiment, the formulation further includes a buffer, such as TRIS. In another preferred embodiment, the formulation comprises a surfactant, such as Brij-35. In an additional preferred embodiment, the GLP-1 molecule of the formulation is an analog of GLP-1 and is selected from the group consisting of a peptide having the amino acid sequence: 
R1-X-Glu-Gly10-Thr-Phe-Thr-Ser-Asp15-Val-Ser-Ser-Tyr-Leu20-Y-Gly-Gln-Ala-
Ala25-Lys-Z-Phe-Ile-Ala30-Trp-Leu-Val-Lys-Gly35-Arg-R2     (SEQ ID NO:2)
 and a pharmaceutically-acceptable salt thereof, wherein R₁ is His or desamino-histidine, X is Ala, Gly or Val, Y is Glu or Gln, Z is Glu or Gln and R₂ is Gly-OH. In an especially preferred embodiement, the GLP-1 molecule is according to SEQ ID NO: 2, wherein R₁ is L-histidine, X is Val, Y is Glu, Z is Glu, and R₂ is Gly-OH. In an alternative preferred embodiment, the GLP-1 molecule of the formulation is a derivative of GLP-1 and is selected from the group consisting of a peptide having the amino acid sequence: 
NH2-His7-Ala-Glu-Gly10-Thr-Phe-Thr-Ser-Asp15-Val-Ser-Ser-Tyr-Leu20-Glu-Gly-
Gln-Ala-Ala25-Lys-Glu-Phe-Ile-Ala30-Trp-Leu-Val-X          (SEQ ID NO:3)
 and a pharmaceutically-acceptable salt thereof, wherein X is selected from the group consisting of Lys and Lys-Gly; a pharmaceutically-acceptable lower alkylester of the peptide; and a pharmaceutically-acceptable amide of the peptide selected from the group consisting of amide, lower alkyl amide, and lower dialkyl amide. In another preferred embodiment, the formulation also comprises a long-acting insulin agent.

The present invention also provides a method of enhancing the expression of insulin in a mammalian pancreatic β-type islet cell in need of such enhancement, comprising administering to the cell, an effective amount of a shelf-stable pharmaceutical formulation, wherein the formulation comprises a therapeutically effective amount of a GLP-1 molecule, a pharmaceutically acceptable preservative, and a tonicity modifier, and wherein the formulation has a pH that is about 8.2 to about 8.8. In a preferred embodiment, the formulation used in the therapeutic method comprises a buffer, such as TRIS. In another preferred embodiment, the formulation used in the therapeutic method further comprises a surfactant, such as Brij-35. In an additional preferred embodiment, the GLP-1 molecule of the formulation thus administered is an analog of GLP-1 and is selected from the group consisting of a peptide having the amino acid sequence: 
R₁-X-Glu-Gly¹⁰-Thr-Phe-Thr-Ser-Asp¹⁵-Val-Ser-Ser-Tyr-Leu²⁰-Y-Gly-Gln-Ala-
Ala²⁵-Lys-Z-Phe-Ile-Ala³⁰-Trp-Leu-Val-Lys-Gly³⁵-Arg-R₂    (SEQ ID NO:2)
 and a pharmaceutically-acceptable salt thereof, wherein R₁ is His or desamino-histidine, X is Ala, Gly or Val, Y is Glu or Gln, Z is Glu or Gln and R₂ is Gly-OH. In an especially preferred embodiment, the GLP-1 molecule administered is according to SEQ ID NO: 2, wherein R₁ is L-histidine, X is Val, Y is Glu, Z is Glu, and R₂ is Gly-OH. In an alternative preferred embodiment, the GLP-1 molecule administered is a derivative of GLP-1 and is selected from the group consisting of a peptide having the amino acid sequence: 
NH2-His7-Ala-Glu-Gly10-Thr-Phe-Thr-Ser-Asp15-Val-Ser-Ser-Tyr-Leu20-Glu-Gly-
Gln-Ala-Ala25-Lys-Glu-Phe-Ile-Ala30-Trp-Leu-Val-X          (SEQ ID NO:3)
 and a pharmaceutically-acceptable salt thereof, wherein X is selected from the group consisting of Lys and Lys-Gly; a pharmaceutically-acceptable lower alkylester of the peptide; and a pharmaceutically-acceptable amide of the peptide selected from the group consisting of amide, lower alkyl amide, and lower dialkyl amide.

The present invention also provides a method of treating diabetes comprising administering to a patient in need of such treatment an effective amount of a shelf-stable pharmaceutical formulation, wherein the formulation comprises a therapeutically effective amount of a GLP-1 molecule, a pharmaceutically acceptable preservative, and a tonicity modifier, and wherein the formulation has a pH that is about 8.2 to about 8.8. In a preferred embodiment, the formulation used in the therapeutic method comprises a buffer, such as TRIS. In another preferred embodiment, the formulation used in the therapeutic method further comprises a surfactant, such as Brij-35. In an additional preferred embodiment, the GLP-1 moleculeof the formulation thus administered is an analog of GLP-1 and is selected from the group consisting of a peptide having the amino acid sequence: 
R1-X-Glu-Gly10-Thr-Phe-Thr-Ser-Asp15-Val-Ser-Ser-Tyr-Leu20-Y-Gly-Gln-Ala-
Ala25-Lys-Z-Phe-Ile-Ala30-Trp-Leu-Val-Lys-Gly35-Arg-R2     (SEQ ID NO:2)
 and a pharmaceutically-acceptable salt thereof, wherein R₁ is His or desamino-histidine, X is Ala, Gly or Val, Y is Glu or Gln, Z is Glu or Gln and R₂ is Gly-OH. In an especially preferred embodiment, the GLP-1 molecule administered is according to SEQ ID NO: 2, wherein R₁ is L-histidine, X is Val, Y is Glu, Z is Glu, and R₂ is Gly-OH. In an alternative preferred embodiment, the GLP-1 molecule administered is a derivative of GLP-1 and is selected from the group consisting of a peptide having the amino acid sequence: 
NH2-His7-Ala-Glu-Gly10-Thr-Phe-Thr-Ser-Asp15-Val-Ser-Ser-Tyr-Leu20-Glu-Gly-
Gln-Ala-Ala25-Lys-Glu-Phe-Ile-Ala30-Trp-Leu-Val-X          (SEQ ID NO:3)
 and a pharmaceutically-acceptable salt thereof, wherein X is selected from the group consisting of Lys and Lys-Gly; a pharmaceutically-acceptable lower alkylester of the peptide; and a pharmaceutically-acceptable amide of the peptide selected from the group consisting of amide, lower alkyl amide, and lower dialkyl amide.

An additional embodiment encompasses a method of providing meal-time glycemic control and basal glycemic control with a single injection comprising administering to a patient in need thereof an effective amount of a shelf-stable pharmaceutical formulation, wherein the formulation comprises a therapeutically effective amount of a GLP-1 molecule, a long acting insulin agent, a pharmaceutically acceptable preservative, and a tonicity modifier, wherein said formulation has a pH that is about 8.2 to about 8.8.

### Detailed Description of the Preferred Embodiments

The present invention provides a shelf-stable formulation of GLP-1, GLP-1 analogs, and GLP-1 derivatives. Because it is known that there are problems with long term stability of GLP-1 as a component in a pharmaceutical composition, the present inventors developed a pharmaceutical formulation which stabilizes GLP-1, its derivatives and analogs. This development led to the shelf-stable GLP-1 formulations of the invention.

In another embodiment, the present invention encompasses a GLP-1 formulation that also comprises a long acting diabetic agent. It has long been a goal of insulin therapy to mimic the pattern of endogenous insulin secretion in normal individuals. The daily physiological demand for insulin fluctuates and can be separated into two phases: (a) the absorptive phase requiring a pulse of insulin to dispose of the meal-related blood glucose surge, and (b) the post-absorptive phase requiring a sustained delivery of insulin to regulate hepatic glucose output for maintaining optimal fasting blood glucose. Accordingly, effective therapy for people with diabetes generally involves the combined use of two types of exogenous insulin formulations: a fast-acting meal time insulin provided by bolus injections and a long-acting, so-called, basal insulin, administered by injection once or twice daily to control blood glucose levels between meals. Examples of commercial basal insulin preparations include NPH (Neutral Protamine Hagedorn) insulin, protamine zinc insulin (PZI), and ultralente (UL).

The term "stability" is used to mean chemical as well as physical stability. Physical stability refers to properties such as protein aggregation, which can be measured by a sample's attenuation of light. The measurement relates to the turbidity of a formulation. Turbidity is produced by aggregation or precipitation of proteins or complexes in the formulation and is indicative of decreased stability of a solution formulation. The more turbid a protein preparation, the less stable the preparation is. Stability also refers to the chemical stability of the formulation such as the propensity of the proteins to form high order polymers which is indicative of decreased stability.

One factor that plays a role in the stability of GLP-1 formulations is the maintenance of pH at a prescribed level. Specifically, the present inventors have found that achieving and maintaining the pH of the formulation at about 8.2 to about 8.8 is advantageous. Typical peptide formulations have a more neutral pH of 7 to about 7.8 or an acidic pH. Furthermore, a composition containing a GLP-1 molecule that has a pH in the range of about 6.8 to about 7.5 exhibits less physical stability than a composition of a GLP-1 molecule containing a preservative and having a pH in the range of about 8.2 to about 8.8. A preserved formulation which has a pH of less than about 8.0 tends to exhibit turbidity, a telltale sign of decreased physical stability of the peptide formulation. Conversely, a formulation which has a pH greater than about 8.8 tends to have decreased chemical stability.

Therefore, the invention contemplates GLP-1 formulations having a pH range of about 8.2 to about 8.8, which preserves optimal chemical and physical stability of the GLP-1 molecule. A particularly preferred range for the inventive GLP-1 formulations is about 8.3 to about 8.6, and a most particularly preferred pH range is about 8.4 to about 8.5. As used in this specification with respect to pH, the term "about" means plus or minus 0.1 pH units. Thus, a pH of "about 8.5" denotes a pH of 8.4 to 8.6.

GLP-1 molecules themselves exhibit a buffering capacity. However, to maintain the pH of the composition for long-term storage and stability, it is preferable to add a buffer.

The choice of buffer affects the chemical and physical stability of the formulation because it influences pH. There are very few pharmaceutically acceptable buffers in the alkaline range. Phosphate buffers, which are typically used in peptide pharmaceutical formulations, cannot maintain a pH range of 8.2 to about 8.8. However, the present inventors have discovered that certain other amine-containing buffers are capable of imparting chemical as well as physical stability to formulations of GLP-1 molecules.

The buffers used in the present invention preferably provide a buffering capacity in the range of about 8.2 to about 8.8. The buffers which are used may be tromethane (TRIS), and amino-acid based buffers such as lysine and hydroxy-lysine. Although any non-phosphate buffer which has a buffering capacity in the range of about 8.2 to about 8.8 may be used, TRIS is the preferred buffer for the formulations of the present invention. The term "TRIS" refers to 2-amino-2-hydroxymethyl-1,3-propanediol (also known in the art as tromethane, trimethylol aminomethane or tris(hydroxymethyl) aminomethane), and to any pharmacologically acceptable salt thereof. The free base and the hydrochloride form are two common forms of TRIS. TRIS is one of the few buffers which is capable of maintaining the pH at the alkaline level desired, thereby stabilizing the formulation.

A second factor that plays a role in the stability of GLP-1 formulations is the concentration of the GLP-1 molecule that is used in the inventive formulation. The present inventors determined that a concentration of about 0.30 to about 0.65 mg/ml of the GLP-1 molecule was stable in the inventive formulations. However, a concentration which was about equal to or greater than 1 mg/ml was unstable. This stability was evidenced by the development of turbidity in the formulation. A particularly stable formulation includes about 0.5 mg/ml of a GLP-1 molecule.

An additional factor which contributes to the overall stability of the GLP-1 formulations of the present invention is the choice of preservative. The preservative is an essential component in the formulation because it enables multiple uses of the formulation. While it is typical that most preservatives would be capable of stabilizing a pharmaceutical formulation, some pharmaceutically acceptable preservatives act to promote physical instability of the formulation. The present inventors have found that a phenolic preservative is preferred. Specifically, the preservative may be m-cresol, phenol, benzyl alcohol, or methyl paraben and is present in an amount from about 2 mg/ml to about 6 mg/ml. Ultimately, the concentration of preservative necessary for effective preservation depends on the preservative used, the pH of the formulation, and whether substances that bind or sequester the preservative are also present. Preferably, m-cresol is used in the formulations as a preservative.

While a buffer and a preservative are most preferably included in the formulation, other additional excipients may be included, such as a tonicity modifier and/or a surfactant as well as distilled water for injections.

The tonicity modifier may be included to make the formulation approximately isotonic with bodily fluid depending on the mode of administration. The concentration of the tonicity modifier is in accordance with the known concentration of a tonicity modifier in a peptide formulation. A preferable tonicity modifier used in the present invention is glycerol.

A surfactant, which may be included in the formulation of the present invention, can be cationic, anionic, or non-ionic. A preferable class of surfactants is polyoxyethylene ethers. A preferred surfactant useful in the present invention is Brij-35, a polyoxyethylene 23 lauryl ether, available from ICI United States, Inc.

The present invention contemplates use of not only natural GLP-1, but also analogs, derivatives and salts of GLP-1. As used herein, the term "a GLP-1 molecule" refers (1) to the naturally-occurring GLP-1, which is GLP-1 (7-37)-OH; (2) GLP-1(7-36)NH₂, as well as (3) GLP-1 (7-37); (4) natural and synthetic functional GLP-1 analogs; (5) derivatives of GLP-1 and (6) salts of any of the aforementioned molecules.

A "GLP-1 analog" is defined as a molecule having one or more amino acid substitutions, deletions, inversions, or additions relative to GLP-1(7-37) and may include the D-amino acid forms. Numerous GLP-1 analogs are known in the art and include, but are not limited to, GLP-1(7-34), GLP-1(7-35), GLP-1(7-36)NH₂, Gln⁹-GLP-1(7-37), d-Gln⁹-GLP-1(7-37), Thr¹⁶-Lys¹⁸-GLP-1(7-37), and Lys¹⁸-GLP-1(7-37), Gly⁸-GLP-1(7-36)NH₂, Gly⁸-GLP-1(7-37)OH, Val⁸-GLP-1(7-37)OH, Met⁸-GLP-1(7-37)OH, acetyl-Lys⁹-GLP-1(7-37), Thr⁹-GLP-1(7-37), D-Thr⁹-GLP-1(7-37), Asn⁹-GLP-1(7-37), D-Asn⁹-GLP-1(7-37), Ser²²-Arg²³-Arg²⁴-Gln²⁶-GLP-1(7-37), Arg²³-GLP-1(7-37), Arg²⁴-GLP-1(7-37), α-methyl-Ala⁸-GLP-1(7-36)NH₂, and Gly⁸-Gln²¹-GLP-1(7-37)OH, and the like.

Other GLP-1 analogs consistent with the present invention are described by the formula: 
R1-X-Glu-Gly10-Thr-Phe-Thr-Ser-Asp15-Val-Ser-Ser-Tyr-Leu20-Y-Gly-Gln-Ala-
Ala25-Lys-Z-Phe-Ile-Ala30-Trp-Leu-Val-Lys-Gly35-Arg-R2 (SEQ ID NO:2)
 wherein: R₁ is selected from the group consisting of L-histidine, D-histidine, desamino-histidine, 2-amino-histidine, beta-hydroxy-histidine, homohistidine, alpha-fluoromethyl-histidine, and alpha-methyl-histidine; X is selected from the group consisting of Ala, Gly, Val, Thr, Ile, and alpha-methyl-Ala; Y is selected from the group consisting of Glu, Gln, Ala, Thr, Ser, and Gly; Z is selected from the group consisting of Glu, Gln, Ala, Thr, Ser, and Gly; and R₂ is selected from the group consisting of NH₂, and Gly-OH.

GLP-1 analogs also have been described in WO 91/11457, and include GLP-1(7-34), GLP-1(7-35), GLP-1(7-36), or GLP-1(7-37), or the amide form thereof, and pharmaceutically-acceptable salts thereof, having at least one modification selected from the group consisting of:
(a) substitution of glycine, serine, cysteine, threonine, asparagine, glutamine, tyrosine, alanine, valine, isoleucine, leucine, methionine, phenylalanine, arginine, or D-lysine for lysine at position 26 and/or position 34; or substitution of glycine, serine, cysteine, threonine, asparagine, glutamine, tyrosine, alanine, valine, isoleucine, leucine, methionine, phenylalanine, lysine, or a D-arginine for arginine at position 36;
(b) substitution of an oxidation-resistant amino acid for tryptophan at position 31;
(c) substitution of at least one of: tyrosine for valine at position 16; lysine for serine at position 18; aspartic acid for glutamic acid at position 21; serine for glycine at position 22; arginine for glutamine at position 23; arginine for alanine at position 24; and glutamine for lysine at position 26; and
(d) substitution of at least one of: glycine, serine, or cysteine for alanine at position 8; aspartic acid, glycine, serine, cysteine, threonine, asparagine, glutamine, tyrosine, alanine, valine, isoleucine, leucine, methionine, or phenylalanine for glutamic acid at position 9; serine, cysteine, threonine, asparagine, glutamine, tyrosine, alanine, valine, isoleucine, leucine, methionine, or phenylalanine for glycine at position 10; and glutamic acid for aspartic acid at position 15; and
(e) substitution of glycine, serine, cysteine, threonine, asparagine, glutamine, tyrosine, alanine, valine, isoleucine, leucine, methionine, or phenylalanine, or the D- or N-acylated or alkylated form of histidine for histidine at position 7;
wherein, in the substitutions described in (a), (b), (d), and (e), the substituted amino acids can optionally be in the D-form and the amino acids substituted at position 7 can optionally be in the N-acylated or N-alkylated form.

Preferred GLP-1 molecules used in the present inventive formulation also include analogs of GLP-1 (7-37)NH₂ and GLP-1 (7-37) in which one or more amino acids which are not present in the original sequence are added or deleted, and derivatives thereof. Specifically, His and desamino-histidine are preferred for R₁. Ala, Gly and Val are preferred at the "X" position. Also, Glu and Gln are preferred for at the "Y" position. Glu and Gln are preferred at the "Z" position and Gly-OH is preferred for R₂.

A particularly preferred GLP-1 analog is known as Val(8)GLP-1 (V8GLP-1) and has a formula according to SEQ ID NO:2, wherein R₁ is L-histidine, X is Val, Y is Glu, Z is Glu and R₂ is Gly-OH.

A "GLP-1 derivative" is defined as a molecule having the amino acid sequence of GLP-1(7-37) or of a GLP-1 analog, but additionally comprises chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the des-amino, N-lower alkyl, N-di-lower alkyl, and N-acyl modifications. Modifications of the terminal carboxy group include, without limitation, the amide, lower alkyl amide, dialkyl amide, and lower alkyl ester modifications. Lower alkyl is C₁-C₄ alkyl. Furthermore, one or more side groups, or terminal groups, may be protected by protective groups known to the ordinarily-skilled protein chemist. The α-carbon of an amino acid may be mono- or dimethylated.

Other GLP-1 derivatives include molecules which are selected from the group consisting of a peptide having the amino acid sequence: 
NH2-His7-Ala-Glu-Gly10-Thr-Phe-Thr-Ser-Asp15-Val-Ser-Ser-Tyr-Leu20-Glu-
Gly-Gln-Ala-Ala25-Lys-Glu-Phe-Ile-Ala30-Trp-Leu-Val-X     (SEQ ID NO:3)
 and pharmaceutically-acceptable salts thereof, wherein X is selected from the group consisting of Lys and Lys-Gly; and a derivative of said peptide, wherein said peptide is selected from the group consisting of: a pharmaceutically-acceptable lower alkylester of said peptide; and a pharmaceutically-acceptable amide of said peptide, selected from the group consisting of amide, lower alkyl amide, and lower dialkyl amide.

Yet other GLP-1 derivatives appropriate for use in the present invention include compounds claimed in U.S. Patent No. 5,512,549 described by the formula: 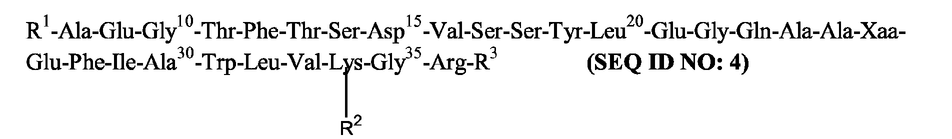 wherein R¹ is selected from the group consisting of 4-imidazopropionyl, 4-imidazoacetyl, or 4-imidazo-α, α dimethyl-acetyl; R² is selected from the group consisting of C₆-C₁₀ unbranched acyl, or is absent; R³ is selected from the group consisting of Gly-OH or NH₂; and, Xaa is Lys or Arg, may be used in present invention.

"DPP-IV protected GLPs" refers to GLP-1 analogs which are resistant to the action of DPP-IV. These include analogs having a modified or D amino acid residue in position 8 and includes biosynthetic GLP-1 analogs having Gly, Val, Thr, Met, Ser, Cys, or Asp in position 8. Other DPP-IV protected GLPs include desamino His⁷ derivatives.

"GLP-1 peptide analogs" are defined as GLP-1 analogs or derivatives which exclude acylated forms.

"Biosynthetic GLP-1 analogs" are defined as any GLP-1 analogs or derivatives which contain only naturally occurring amino acid residues and are thus capable of being expressed by living cells, including recombinant cells and organisms.

Methods for preparing the GLP-1 molecules which are incorporated into the shelf stable formulations of the invention are well known to the skilled artisan. In one method, GLP-1 molecules are prepared by well-known methods of peptide synthesis, such as those described in Merrifield, (*Chem. Soc.* Vol. 85, page 2149, 1962). It is also contemplated that the molecules can be prepared by fragmenting the natural amino acid sequence of GLP-1(7-37) with, for example, a proteolytic enzyme. It is also contemplated that recombinant DNA techniques can be used to prepare the molecules, such as the methods of Maniatis *et al. (Molecular Biology: A Laboratory Manual,* CSH, 1982).

Administration may be via any route known to be effective by the physician of ordinary skill. Parenteral administration is preferred. Parenteral administration is commonly understood as administration by other than a gastro-intestinal route. Preferred parenteral routes for administering the formulations of the present invention include intravenous, intramuscular, subcutaneous, intraperitoneal, intraarterial, nasal, pulmonary, and buccal routes. Intravenous, intraperitoneal, intramuscular, and subcutaneous routes of administration of the compounds used in the present invention are more preferred parenteral routes of administration. Intravenous, intraperitoneal, and subcutaneous routes of administration of the formulations of the present invention yet more highly preferred. The most preferred route of administration is via a pen injection system, wherein either a 1.5 ml cartridge or a 3.0 ml cartridge is utilized.

Administration via certain parenteral routes may involve introducing the formulations of the present invention into the body of a patient through a needle or a catheter, propelled by a sterile syringe or some other mechanical device such as an continuous infusion system. A formulation provided by the present invention may be administered using a syringe, injector, pump, or any other device recognized in the art for parenteral administration. A formulation of the present invention may also be administered as an aerosol for absorption in the lung or nasal cavity. The formulations may also be administered for absorption through the mucus membranes, such as in buccal administration.

The amount of a formulation of the present invention that is administered to treat diabetes or hyperglycemia depends on a number of factors, among which are included, without limitation, the patient's sex, weight and age, the underlying causes of the condition or disease to be treated, the route of administration and bioavailability, the persistence of the administered GLP-1, its analog, or its derivative in the body, the formulation, and the potency of the GLP-1 molecule. Where administration is intermittent, the amount per administration should also take into account the interval between doses, and the bioavailability of the GLP-1 molecule from the formulation. Administration of the formulation of the present invention could be continuous. It is within the skill of the ordinary physician to titrate the dose and infusion rate or frequency of administration of the formulation of the present invention to achieve the desired clinical result.

The formulations of the present invention have insulinotropic activity. Thus, another aspect of the present invention provides a method of enhancing the release of insulin from pancreatic islet cells comprising providing to a mammalian pancreatic β-type islet cell the inventive formulation which contains an effective amount of GLP-1, a GLP-1 analog, a GLP-1 derivative or a salt thereof.

That the present invention provides formulations of GLP-1, its analogs, and its derivatives having greatly increased physical and chemical stability relative to peptide formulations the art will be readily apparent from the following data and examples.

The following examples and preparations are provided merely to further illustrate the preparation of the formulations of the invention. The scope of the invention is not limited to the following examples.

### Example 1-Preparation and chemical stability testing

66.2 mg of Val(8) GLP-1 was dissolved in water at 1.0 mg/ml and adjusted to pH 8.51. Three formulations were made as follows:
(A) A 21.5 ml aliquot of peptide solution in water was mixed with 21.5 ml of 0.63% m-cresol-3.2% glycerol and the final pH was set to 8.48. The solution was passed thru a 0.2 micron filter. Then aliquots of the solution, containing 0.5 mg/ml peptide in 0.315% m-cresol-1.6% glycerol at pH 8.48, were pipetted into parenteral vials and stoppered.
(B) A 21.5 ml aliquot of peptide solution in water was mixed with 21.5 ml of 0.63% m-cresol-3.2% glycerol- 0.02 molar L-Lysine pH 8.5 and the final pH was set to 8.48. The solution was passed thru a 0.2 micron filter. Then, aliquots of the solution, containing 0.5 mg/ml peptide in 0.315% m-cresol-1.6% glycerol- 0.01 molar L-Lysine at pH 8.48, were pipetted into parenteral vials and stoppered.
(C) A 21.5 ml aliquot of peptide solution in water was mixed with 21.5 ml of 0.63% m-cresol-3.2% glycerol- 0.02 molar Tris buffer pH 8.5 and the final pH was set to 8.50. The solution was passed thru a 0.2 micron filter. Aliquots of the solution, containing 0.5 mg/ml peptide in 0.315% m-cresol-1.6% glycerol- 0.01 molar tris at pH 8.50, were pipetted into parenteral vials and stoppered.

One set of vials was held in the refrigerator at 4°C. A second set was held at 30° C (room temperature). A third set was held in the refrigerator, but was taken out three times a day, 5 days a week, and allowed to warm up to room temperature. Then the set was put back in the refrigerator.

At various time points, vials of the three formulations at the three temperature conditions were examined visually, checked with a pH meter, and assayed for main peak purity and total mg/ml of peptide by HPLC. Before assaying on HPLC, the vialed material was spun in centrifuge; the supernatant was injected on the column. The concentration of a freshly prepared solution of Val(8) GLP-1 in 0.01 molar HCl was determined by UV spectroscopy. Standards of known concentration were prepared by diluting this solution; these were injected on HPLC to get a multipoint standard curve. The main peak purity was determined by comparing the main peak area to the total area. The total area (main peak plus related substances) was converted to total mg/ml using the standard curve parameters.

The Table below summarizes a chemical stability study using HPLC to quantify potency, showing that the formulation is stable when there is not a significant loss peptide from solution. %purity is a measure of the stability of the formulation. Chemical degradation of the peptide to soluble related substances results in a lower purity value. The results in this Table show that GLP-1 in TRIS buffer had the highest purity, and hence the highest stability values.

**Table 1**

| Amount of GLP-1 | % Purity at 4° | % Purity with starting Temp at 4° with warming to 30° | % Purity at 30° | Buffer added |
|---|---|---|---|---|
| 0.4849 | 98.13 | 98.13 | 98.13 | No buffer |
| 0.4795 | 98.52 | | | No buffer |
| 0.4636 | | 98.26 | | No buffer |
| 0.4829 | | | 95.55 | No buffer |
| 0.4801 | 98.49 | | | No buffer |
| 0.4674 | | 98.43 | | No buffer |
| 0.4544 | | | 94.14 | No buffer |
| 0.4327 | 97.20 | | | No buffer |
| 0.3914 | | 97.82 | | No buffer |
| 0.3846 | | | 87.741 | No buffer |
| 0.4567 | 98.27 | 98.27 | 98.27 | 0.01M lysine |
| 0.4280 | 98.47 | | | 0.01 Lysine |
| 0.4293 | | 98.11 | | 0.01 Lysine |
| 0.4245 | | | 94.90 | 0.01 Lysine |
| 0.4539 | 98.58 | | | 0.01 Lysine |
| 0.4016 | | 98.55 | | 0.01 Lysine |
| 0.4338 | | | 93.68 | 0.01 Lysine |
| 0.3923 | 96.93 | | | 0.01 Lysine |
| 0.3922 | | 96.84 | | 0.01 Lysine |
| 0.4034 | | | 85.93 | 0.01 Lysine |
| 0.4533 | 98.37 | 98.37 | 98.37 | 0.01 TRIS |
| 0.4602 | 98.52 | | | 0.01 TRIS |
| 0.4340 | | 98.47 | | 0.01 TRIS |
| 0.4374 | | | 97.68 | 0.01 TRIS |
| 0.4709 | 98.71 | | | 0.01 TRIS |
| 0.4531 | | 98.53 | | 0.01 TRIS |
| 0.4377 | | | 96.72 | 0.01 TRIS |
| 0.4569 | 97.67 | | | 0.01 TRIS |
| 0.4354 | | 97.81 | | 0.01 TRIS |
| 0.4170 | | | 92.13 | 0.01 TRIS |

### Example 2-Preparation and Physical Stability Testing

A solution of Val(8) GLP-1 in water was adjusted to pH 8.50 and filtered. The concentration was determined to be 5.28 mg/ml by UV analysis. Aliquots containing 5.00 mg of peptide were pipetted into 10 ml parenteral vials and freeze dried. After freeze drying, the plugs were reconstituted by adding 10.00 ml of either:
a) 0.315% m-cresol-1.6% glycerol pH 8.5
b) 0.315% m-cresol-1.6% glycerol- 0.005molar Tris buffer pH 8.5
c) 0.315% m-cresol-1.6% glycerol- 0.01 molar Tris buffer pH 8.5

Turbidity measurements were measured initially and after 1 month in the refrigerator. All turbidities for the samples (at room temperature) were less than 10 NTU. The initial measurements were made on a Hach 2100N Turbidimeter while the 1 month readings were made on a Hach 2100 AN Turbidimeter.

| Buffer | Turbitity (Initial) | Turbidity (1 month) |
|---|---|---|
| none | 0.4 NTU | 4.0 NTU |
| .005 molar Tris | 0.4 NTU | 3.6 NTU |
| .010 molar Tris | 0.4 NTU | 4.5 NTU |

## Claims

1. A shelf-stable pharmaceutical formulation comprising a therapeutically effective amount of a GLP-1 molecule, a pharmaceutically acceptable preservative, and a tonicity modifier, wherein said formulation has a pH that is 8.2 to 8.8.

2. The formulation of claim 1, further comprising a buffer.

3. The formulation of claim 2, wherein the buffer is TRIS.

4. The formulation of any one of claims 1 to 3, further comprising a surfactant.

5. The formulation of claim 4, wherein the surfactant is Brij-35.

6. The formulation of any one of claims 1 to 5, wherein the GLP-1 molecule is an analog of GLP-1 and is selected from a peptide having the amino acid sequence: 
R1-X-Glu-Gly10-Thr-Phe-Thr-Ser-Asp15-Val-Ser-Ser-Tyr-Leu20-Y-Gly-Gln-Ala-
Ala25-Lys-Z-Phe-Ile-Ala30-Trp-Leu-Val-Lys-Gly35-Arg-R2     (SEQ ID NO:2)
 or a pharmaceutically-acceptable salt thereof, wherein R₁ is His or desamino-histidine, X is Ala, Gly or Val, Y is Glu or Gln, Z is Glu or Gln and R₂ is Gly-OH.

7. The formulation of claim 6, wherein R₁ is L-histidine, X is Val, Y is Glu, Z is Glu, and R₂ is Gly-OH.

8. The formulation of any one of claims 1 to 5, wherein the GLP-1 molecule is a derivative of GLP-1 and is selected from a peptide having the amino acid sequence: 
NH2-His7-Ala-Glu-Gly10-Thr-Phe-Thr-Ser-Asp15-Val-Ser-Ser-Tyr-Leu20-Glu-Gly-
Gln-Ala-Ala25-Lys-Glu-Phe-Ile-Ala30-Trp-Leu-Val-X          (SEQ ID NO:3)
 or a pharmaceutically-acceptable salt thereof, wherein X is selected from Lys and Lys-Gly; a pharmaceutically-acceptable lower alkylester of said peptide; and a pharmaceutically-acceptable amide of said peptide selected from amide, lower alkyl amide, and lower dialkyl amide.

9. The formulation of any one of claims 1 to 8 for use as a medicament.

10. The use of a formulation of any one of claims 1 to 8 for the manufacture of a medicament for the enhancement of expression of insulin in a mammalian pancreatic β-type islet cell.

11. The use of a formulation of any one of claims 1 to 8 for the manufacture of a medicament for the treatment of diabetes.

12. The formulation of any one of claims 1 to 8, further comprising a long-acting insulin agent.

13. The use of the formulation of claim 12 for the manufacture of a medicament for meal-time glycemic control and basal glycemic control with a single injection.
